# EUROPEAN PATENT SPECIFICATION

(11) **EP 3 517 020 B1**
(45) Date of publication and mention of the grant of the patent: **25.01.2023**
(21) Application number: 17853145.5
(22) Date of filing: 21.09.2017
(51) Int. Cl.: A61B 3/10

(54) **SCANNING LASER OPHTHALMOSCOPE**
ABTASTLASER-OPHTHALMOSKOP
OPHTALMOSCOPE LASER Á BALAYAGE

(30) Priority: 21.09.2016 JP 2016184659
(43) Date of publication of application: 31.07.2019
(73) Proprietor: Tomey Corporation, Nagoya-shi, Aichi-ken, 451-0051 (JP); QD Laser, Inc., Kawasaki-shi, Kanagawa 210-0855 (JP)
(72) Inventor: SUGAWARA, Mitsuru, Kawasaki-shi Kanagawa 210-0855 (JP); SUZUKI, Makoto, Kawasaki-shi Kanagawa 210-0855 (JP); MUKAI, Hideo, Nagoya-shi Aichi 451-0051 (JP)
(74) Representative: Winter, Brandl - Partnerschaft mbB
(86) International application number: PCT/JP2017/034163
(87) International publication number: WO 2018/056377

(56) References cited:
- JP-A- H10 272 098
- JP-A- H10 272 098
- JP-A- 2015 111 231
- JP-A- 2015 111 231
- US-A- 5 430 509
- US-A1- 2002 036 750
- US-A1- 2013 194 548
- US-A1- 2016 089 016
- US-A1- 2016 166 146

## Description

### Technical Field

The technique disclosed herein relates to a scanning laser ophthalmoscope (SLO) configured to capture a frontal image of an eye of a subject.

### Background Art

In the field of ophthalmology, a scanning laser ophthalmoscope is known which obtains a fundus image by scanning laser light on a fundus of an eye of a subject in a two-dimensional direction (for example, Japanese Patent Application Publication No. H11-197109). The related prior art is disclosed in US 2016/089016 A1, which discloses a fundus photographing apparatus with wavefront compensation. Further prior art is also disclosed in US 5 430 509 A.

### Summary

Many ophthalmologic devices, including such an ophthalmoscope as above, are provided with a means for contacting a head of a subject to hold an eye of the subject at a predetermined position with respect to an optical system for ocular examination or image capturing.

However, since the above-mentioned contacting means is not configured to completely fix the head of the subject with respect to the device, the eye of the subject (the head of the subject) may move during the ocular examination or image capturing. In this case, the ocular examination or the image capturing may not be performed appropriately and may end abnormally.

For this reason, an increasing number of devices employ an alignment mechanism that enables an optical system for ocular examination or image capturing to follow up movements of the eye of the subject. However, the follow-up range is limited, and thus the devices are not capable of coping with the movements beyond this allowable range. Besides, providing the alignment mechanism makes the devices complicated, which is problematic in downsizing the devices.

The disclosure herein discloses a scanning laser ophthalmoscope which is capable of avoiding a failure that an ocular examination or image capturing cannot be appropriately completed due to significant deviation of an eye of a subject from a reference position set in the device as well as is capable of achieving downsizing by eliminating a need for an alignment mechanism.

A scanning laser ophthalmoscope disclosed herein comprises the features of claim 1.

With such a configuration, the guide mirror configured to guide the laser light to the fundus of the eye of the subject can be held at a predetermined position with respect to the eye of the subject. In addition, since the scanner configured to scan the laser light is disposed at the guide mirror holder, the scanner can be held in a predetermined positional relationship with the eye of the subject. Therefore, the eye of the subject is prevented from largely deviating from a reference position, and hence, an ocular examination or image capturing can be performed appropriately. Besides, since the guide mirror holder holds the positional relationship between the eye of the subject and the scanner, there is no need for an alignment mechanism for following up movements of the eye of the subject, and thus downsizing can be achieved.

The guide mirror holder comprises a structure configured to be fixed to a head of the subject.

With such a structure, the guide mirror and the scanner are fixed to the head of the subject by the guide mirror holder, by which the eye of the subject and each of the guide mirror and the scanner can be held in a substantially constant positional relationship without being influenced by movement of the head of the subject.

Further, in the scanning laser ophthalmoscope disclosed herein, the guide mirror may comprise a free-form surface or a combined structure of a free-form curve and a diffraction surface.

With such a configuration, the guide mirror enables the laser light scanned by the scanner to enter the eye of the subject at an appropriate angle.

Further, in the scanning laser ophthalmoscope disclosed herein, the light source may be configured to be capable of simultaneously or selectively emitting first laser light and second laser light, the first laser light comprising a wavelength in an infrared range used in acquiring the fundus image of the eye of the subject, the second laser light comprising a wavelength in a visible range, and the fundus of the eye of the subject being irradiated by the second laser light such that a photogene of the second laser light is recognized as a fixation target in a form of figure.

With such a configuration, the fundus image of the eye of the subject can be obtained by using the first laser light having the wavelength in the infrared range and the eye of the subject can recognize the fixation target by using the second laser light having the wavelength in the visible range. Due to this, the movement of the eye can be suppressed during image acquisition and a stable image can thereby be obtained.

### Brief Description of Drawings

FIG.1A is a top view of a scanning laser ophthalmoscope according to an embodiment, and FIG. 1B is a side view thereof.
FIG.2 is a block diagram showing a schematic configuration of the scanning laser ophthalmoscope according to the embodiment.
FIG.3 is a diagram showing a configuration of a light source used in the scanning laser ophthalmoscope according to the embodiment.
FIG.4 is a diagram showing a flow as to driving of the light source and a scanning mechanism according to the embodiment.
FIG.5 is a diagram showing an example of a scanning trajectory of laser light in raster scanning by a scanning mirror.
FIG.6 is a diagram showing a relationship between a scanning area and a target presentation area.
FIG. 7 is a diagram showing another example of a scanning trajectory of the laser light in raster scanning by the scanning mirror.

### Detailed Description

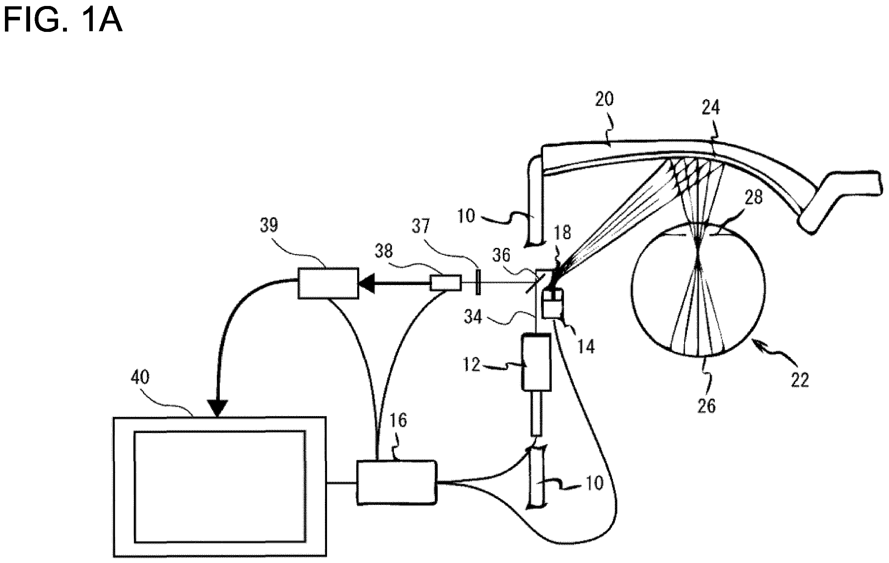

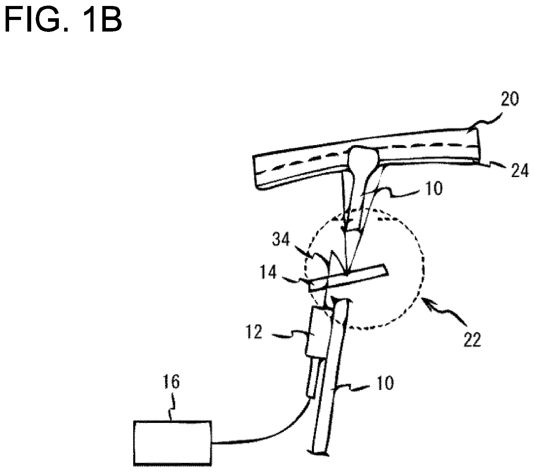

Hereinafter, a scanning laser ophthalmoscope according to an embodiment will be described. As shown in FIGS. 1A and 1B, a temple 10 of eyewear that holds a guide mirror 24 and an eyewear lens 20 is provided with a light source 12 configured to emit laser light 34 and a scanning mirror 14 which is a scanner configured to scan the laser light 34 emitted from the light source 12 in a two-dimensional direction. An infrared light transmission filter 37, a light receiving sensor 38, and an image processor 39 are provided at an external device (not shown) which is separated from the temple 10 (i.e., the eyewear). The light source 12 is configured to emit a plurality of laser light with different wavelengths. In FIG. 1B, the illustration of the laser light 34 is partly omitted for clarity of the figure.

A controller 16 is configured to control the emission of the laser light 34 from the light source 12. The controller 16 may be provided on the temple 10 of the eyewear similarly to the light source 12 and the scanning mirror 14 described above, or may be provided at an external device separated from the eyewear. Here, a case where the controller 16 is provided at the external device (not shown) will be described as an example. The light source 12 and the controller 16 provided at the external device are electrically connected to each other, for example, by a cable (not shown).

The laser light 34 emitted from the light source 12 is guided to the scanning mirror 14. A split mirror 36 is disposed on a path of the laser light 34 emitted from the light source 12. The split mirror 36 is provided on the temple 10 of the eyewear. A lens and a mirror for causing the laser light 34 to enter the scanning mirror 14 are provided between the light source 12 and the split mirror 36, but the illustration for these lens and mirror is omitted.

The scanning mirror 14 is configured to scan the laser light 34 emitted from the light source 12 such that the laser light 34 is directed two-dimensionally toward a retina 26 located at a fundus of an eye 22 of a subject. The scanning mirror 14 is, for example, a Micro Electro Mechanical Systems (MEMS) mirror and is configured to scan laser light horizontally and vertically.

The laser light 34 (scan light) scanned by the scanning mirror 14 is reflected by a mirror 18 provided on the temple 10 of the eyewear to be directed towards the eyewear lens 20. The guide mirror 24 is provided on a surface of the lens 20 on a subject's eye 22 side. The guide mirror 24 guides the laser light 34 (scan light) scanned by the scanning mirror 14 to the retina 26 of the eye 22. The guide mirror 24 may comprise, for example, a free-form surface or a combined structure of a free-form surface and a diffraction surface. By providing the guide mirror 24 with a free-form surface, the laser light 34 that entered the guide mirror 24 can be converged at a same point (a point between a pupil 28 and the retina in FIGS. 1A and 1B). As shown in FIGS. 1A and 1B, since the laser light 34 is scanned by the scanning mirror 14, it enters the guide mirror 24 at different positions. Thus, with the guide mirror 24 comprising the free-form surface (surface whose curvature changes), the light reflected at different positions of the guide mirror 24 can be converged at a same point. In addition, by providing a surface of the guide mirror 24 with a diffraction surface on which minute concavities and convexities are provided, a phase of a wavefront of the laser light 34 can be controlled, and reflection angles thereof at the guide mirror 24 can thereby be controlled. Therefore, with the guide mirror 24 comprising the combined structure of the free-form surface and the diffraction surface (curved surface on which concavities and convexities are provided), reflection angles at the guide mirror 24 can be appropriately adjusted and the laser light 34 can be converged at a desired point.

The laser light 34 guided to the retina 26 is reflected by the retina 26, and reversely travels toward the light source 12 along the same path as the one it travelled along when guided. A part of a luminous flux of the reflected light has its traveling direction changed at the split mirror 36 described above and is guided to the light receiving sensor 38 through the infrared light transmission filter 37. Here, a luminous flux in a visible range is eliminated by the infrared light transmission filter 37. This is because the luminous flux in the visible range is disturbance to a luminous flux in an infrared range since it is supposed to be used to fixate the subject's eye 22 and it illuminates only in a part of a scan area. The image processor 39 generates an image from the reflected light from the retina 26 received by the light receiving sensor 38 and the image is displayed on a monitor 40.

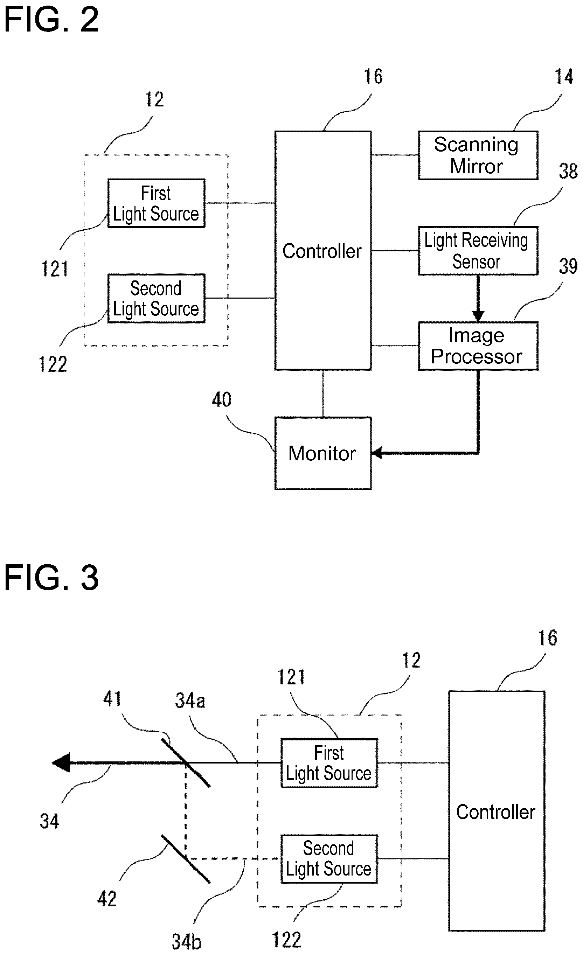

FIG. 2 shows a block diagram of the scanning laser ophthalmoscope shown in FIGS. 1A and 1B. The scanning laser ophthalmoscope of the present embodiment irradiates the subject's eye with a luminous flux for acquiring a fundus image of the subject's eye and a luminous flux for fixating the subject's eye in a predetermined direction. In the present embodiment, the scanning laser ophthalmoscope includes a first light source 121 configured to emit the luminous flux used to acquire the fundus image, and a second light source 122 configured to emit the luminous flux used to fixate the subject's eye. That is, the light source 12 includes the first light source 121 and the second light source 122, and the laser light 34 is emitted by the light sources 121, 122. The first light source 121 is configured to emit a laser luminous flux in the infrared range, and the second light source 122 is configured to emit a laser luminous flux in the visible range.

Here, the laser light 34 emitted from the light source 12 will be described in more detail. FIG. 3 shows an optical configuration in which the luminous fluxes emitted from the first light source 121 and the second light source 122 are directed toward the scanning mirror 14. Laser light 34a emitted from the first light source 121 and laser light 34b emitted from the second light source 122 are made coaxial with each other by a cold mirror 41 and guided to the scanning mirror 14 as the laser light 34. Although a mirror 42 is provided on an optical path of the laser light 34b, the mirror 42 can be omitted by devising an arrangement of the second light source 122 with respect to the first light source 121.

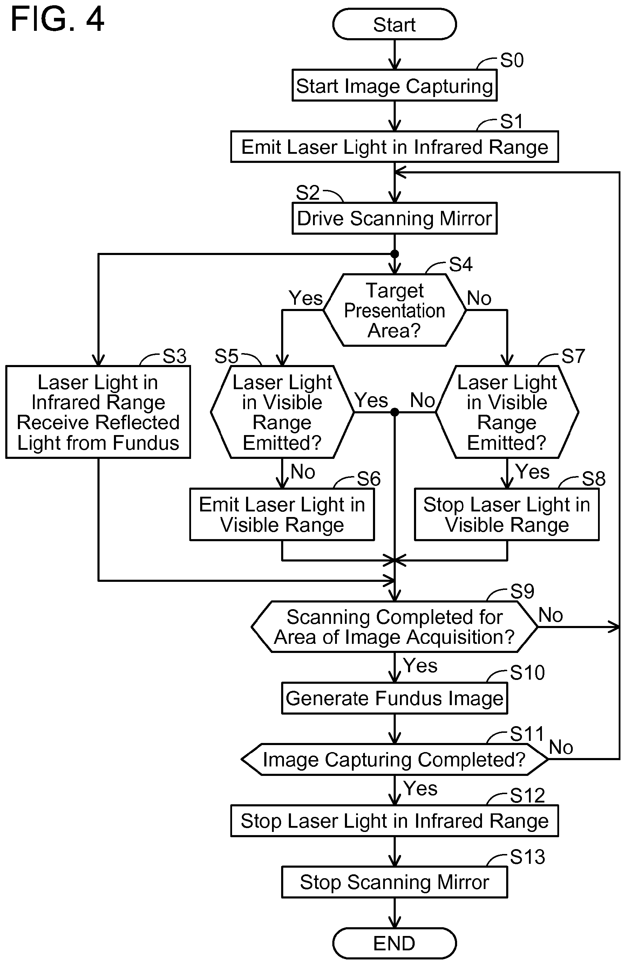

Next, a series of operations of the scanning laser ophthalmoscope according to the present embodiment will be described. FIG. 4 shows an outline of a flow as to driving of laser light and a scanning mechanism in capturing a fundus image during ocular fixation of the subject.

In S0, in response to input of an image-capture start signal through an image-capture button (not shown) or the like, the device shifts to an image-capture mode. When shifting to the image-capture mode, the device irradiates the subject's eye 22 with the laser light 34a for acquiring a fundus image of the subject's eye 22. Specifically, in S1, the controller 16 causes the first light source 121 included in the light source 12 to emit light, by which the subject starts to be irradiated with the laser light 34a.

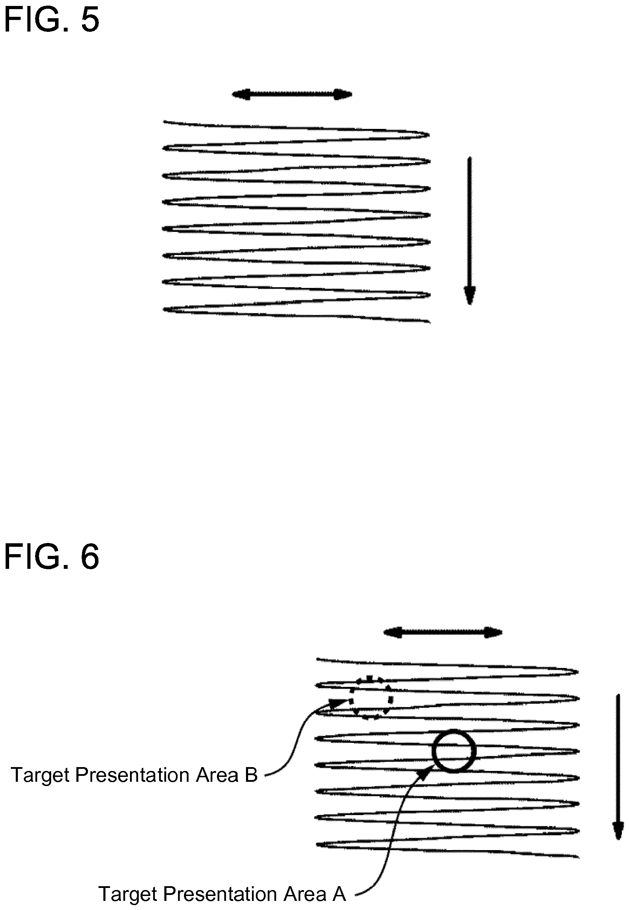

Following the irradiation start of the laser light 34a, the scanning mirror 14 starts to be driven in S2. As a result, the retina 26 of the subject is irradiated with the laser light 34a via the guide mirror 24. Here, in a case where raster scanning is employed as a driving method for the scanning mirror 14, the laser light 34a is scanned to have a trajectory on the retina 26, for example, as shown in FIG. 5. In this example, the light is scanned repeatedly in left-right directions with an upper left end of the trajectory as a starting point, and its scanning position is moved downward over time. In the example shown here, the amount of downward movement is emphasized to make the trajectory clearly understood, and thus an area on which the light is actually scanned may be considered small. However, the amount of downward movement per one scanning in the left-right directions is actually about a line width of the trajectory, and as a result, the scanning mirror 14 is controlled to be driven such that the light is scanned over a rectangular area from its upper left to lower right with almost no gap. An example of such driving is a reciprocating driving mechanism, such as a galvano mirror.

The laser light 34a that has reached the retina 26 of the subject is reflected by the retina 26 and travels reversely along the same path toward the light source 12. A part of that light is guided to the light receiving sensor 38 by the split mirror 36. Since the laser light 34a is in the infrared range, it passes through the infrared light transmission filter 37 provided immediately before the light receiving sensor 38 and then reaches the light receiving sensor 38. The matters described here corresponds to S3 in the flow, and a fundus image is generated as shown by arrows in FIG. 2.

Next, a procedure for suppressing movements of the subject's eye 22 while it is irradiated with the laser light 34a will be described.

As a conventional method of suppressing the movements of the subject's eye 22, a target is presented in a visual field of the subject to fixate the visual of the target. In a conventional method of presenting a target, which is widely used in ophthalmologic devices, a target plate which is arranged coaxially with a measurement optical axis of a device is illuminated with visible light and a subject is caused to visually recognize it. However, this configuration requires separately preparing an optical system for presenting the target, which makes the configuration complicated and is disadvantageous in downsizing the device.

In view of this, in the present embodiment, the optical system for acquiring the fundus image is used as it is to present a fixation target to the subject. In this method, a photogene of a luminous flux scanned on the retina 26 by high-speed scanning is recognized as the target.

As described above, the scanning laser ophthalmoscope according to the present embodiment comprises the configuration that emits the laser light 34b in the visible range separately from the laser light 34a in the infrared range used for acquiring the fundus image. Further, the laser light 34b is emitted toward the subject's eye 22 coaxially with the laser light 34a. However, since the laser light 34b is emitted from the second light source 122 different from the first light source 121 that emits the laser light 34a, the emission of the laser light 34b can be controlled independently of the laser light 34a.

The laser light 34a is emitted to an entirety of the scan area until acquisition of the fundus image is completed, but the laser light 34a is not visually recognized by the subject because it is in the infrared range. When the laser light 34b is emitted while the laser light 34a is emitted, the laser light 34b, which is the luminous flux in the visible range, is visually recognized by the subject. However, if the laser light 34b is emitted to the entirety of the scan area similarly to the laser light 34a, the entire visual field of the subject is illuminated, as a result of which the subject's eye cannot be fixated in a predetermined direction. Therefore, the emission of the laser light 34b needs to be controlled such that it is emitted when an area where the laser light 34a is scanned coincides with an area to which the subject is to be fixated.

For this reason, in S4, it is determined whether or not a position of the luminous flux that is scanned by the scanning mirror 14 and directed toward the fundus of the subject corresponds to a position of an area that presents the target to the subject. Here, a specific determination procedure will be described with reference to FIG. 6.

FIG. 6 is a diagram in which a position at which a fixation target is presented is associated with the scan area shown in FIG. 5. A target presentation area A shown in a center of FIG. 6 indicates an area irradiated with the laser light 34b when a circular target is to be presented near a center of the visual field of the subject in the scan area of the laser light 34. Here, when the target presentation area A is determined, a scanning condition of the scanning mirror 14 for irradiating the target presentation area A with the laser light 34b is determined in a designed manner. Therefore, by continuously monitoring a scanning state of the scanning mirror 14, the controller 16 determines whether or not the laser light 34 is scanned to irradiate the target presentation area A.

In a case where it is determined that the laser light 34 is scanned to irradiate the target presentation area A in S4, the flow proceeds to S5 to check whether or not the laser light 34b in the visible range is emitted. At this time, in a case where the laser light 34b is emitted, the flow proceeds to S9. In FIG. 6, this situation is when the laser light 34 is scanned along the trajectory inside the target presentation area A.

However, in a case where the laser light 34b is not emitted in S5, the flow proceeds to S6 to emit the laser light 34b, and then the flow proceeds to S9. The situation at this time is when the laser light 34 is scanned on a left-side boundary of the target presentation area A in FIG. 6.

In a case where it is determined that the laser light 34 is not scanned to irradiate the target presentation area A in S4, the flow proceeds to S7 to check whether or not the laser light 34b in the visible range is emitted. At this time, in a case where the laser light 34b is not emitted, the flow proceeds to S9. In FIG. 6, this situation is when the laser light 34 is scanned along the trajectory outside the target presentation area A.

However, in a case where the laser light 34b is emitted in S7, the flow proceeds to S8 to stop emission of the laser light 34b, and then the flow proceeds to S9. The situation at this time is when the laser light 34 is scanned on a right-side boundary of the target presentation area A in FIG. 6.

When the flow reaches S9 through the above-described steps, the scanning state for an area for which the fundus image is generated is checked. At this time, in a case where it is determined that the scanning has been completed, the flow proceeds to S 10 to generate the fundus image, however, in a case where it is determined that the scanning is not completed yet, the flow returns to S2 to receive the reflected light by the fundus at a next scanning position.

After the fundus image is generated in S10, whether or not the image capturing has been completed is checked in S11. At this time, in a case where it is determined that the image capturing is not completed yet, the flow returns to S2, and a new round of image capturing is started to acquire a new image.

However, in a case where it is determined that the image capturing has been completed in S11, the flow proceeds to S12 to stop emission of the laser light 34a in the infrared range used for image acquisition. Thereafter, in S13, the driving of the scanning mirror 14 is stopped, whereby the acquisition of the fundus image is completed.

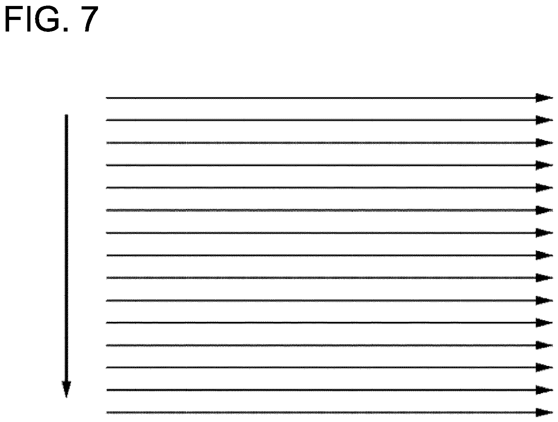

The scanning mirror 14 may be controlled to be driven to cause the laser light 34 to have a trajectory shown in FIG. 7. In this example, lateral scanning is performed only in one direction from left to right, and during the lateral scanning, no movement is made in an up-down direction. Then, after the lateral scanning is performed once, a scanning position is newly set at the left end for the lateral scanning and is further moved in the up-down direction by a predetermined amount, and then the next lateral scanning is performed. As compared to the scanning shown in FIG. 5 in which intervals of the trajectory in the up-down direction near right and left ends and a center of the trajectory cannot be set constant, the scanning shown in FIG. 7 is superior because the trajectory intervals in the up-down direction can be set constant and that makes it possible to avoid differences in amount of information depending on areas. An example of such driving includes a mechanism in which the lateral scanning is performed by a rotating body such as a polygon mirror and the scanning in the up-down direction is performed by a reciprocating scanning mirror, or the like.

In the scanning laser ophthalmoscope of the present embodiment, the scanning mirror 14 is disposed on the temple 10 of the eyewear worn by the subject, and the laser light 34 scanned by the scanning mirror 14 is guided to the retina 26 of the subject by the guide mirror 24 provided on the eyewear lens 20. Therefore, a positional relationship between the scanning mirror 14, the guide mirror 24, and the subject's eye 22 can be maintained constant. Thus, there is no need for an alignment mechanism for following movements of the subject's eye, by which downsizing of the device can be achieved. In addition, with the configuration similar to an eyewear-type wearable terminal, the subject does not need to contact its head on a chin rest or the like upon the fundus image acquisition, by which burden on the subject can be reduced.

In the embodiment described above, the light source 12 and the scanning mirror 14 are provided on an outer side of the temple 10 of the eyewear, however, they may be provided on an inner side of the temple 10 by widening a width of the temple 10 of the eyewear. Furthermore, in the embodiment, the light source 12 is provided on the temple 10 of the eyewear, however, the light source 12 may be provided separately from the eyewear. The temple 10 of the eyewear, which is an example of "guide mirror holder", comprises a configuration fixable to the head of the subject, by which the positional relationship between the guide mirror 24 and the subject's eye 22 can be maintained constant.

Further, in the embodiment described above, the target presentation area A that directs the subject's eye 22 to the vicinity of the center of the visual field is used as shown in FIG. 6, however, the target presented to the subject is not necessarily set to be at the center of the visual field. For example, the target can be set in a peripheral portion of the visual field as shown by a target presentation region B indicated by a broken line at an upper left of FIG. 6. In this case, the subject's eye 22 can be tilted relative to an image-capture optical axis, as a result of which a fundus image for an area different from the area in case of the frontal fixation can be obtained.

In addition, although the scanning mirror 14 (e.g., a MEMS mirror) is exemplified as the scanner configured to scan laser light two-dimensionally, other configuration such as potassium tantalate niobate (KTN) crystal, which is an electro-optical material, may be used as long as it can scan laser light two-dimensionally. Although the case where an image is projected onto the retina 26 of one eye 22 among two eyes is described above as an example, the technique disclosed herein can be applied to a case where fundus images of both eyes 22 are generated.

In the present embodiment, the positional relationship between the scanning mirror 14, the guide mirror 24, and the subject's eye 22 is maintained constant by employing the configuration similar to that of an eyewear-type wearable terminal, however, the technique disclosed herein is not limited thereto. For example, this technique can also be applied to a desktop device such as a conventional scanning laser ophthalmoscope. In this case, a positional relationship between the device and a subject's eye can be maintained constant by providing a configuration that fixes the head of the subject with respect to the device (e.g., a fixing band). Due to this, there is no need for the alignment mechanism for following movements of the subject's eye and downsizing of the device can be achieved.

While specific examples of the present disclosure have been described above in detail, these examples are merely illustrative and place no limitation on the scope of the patent claims. The technology described in the patent claims also encompasses various changes and modifications to the specific examples described above.

## Claims

1. A scanning laser ophthalmoscope comprising:
a light source (12) configured to emit laser light (34);
a scanner (14) configured to scan the laser light (34) emitted from the light source (12) two-dimensionally;
a guide mirror (24) configured to guide the laser light (34) scanned by the scanner (14) to a fundus of an eye (22) of a subject;
a light receiver (38) configured to receive reflected light of the laser light (34) reflected on the fundus; and
an image generator (39) configured to generate a fundus image based on the reflected light received by the light receiver (38),
wherein
the guide mirror (24) is disposed on a path connecting the scanner (14) and the fundus of the eye (22) of the subject, and disposed in front of the eye (22) of the subject
**characterised by**
a guide mirror holder (10) comprising a structure configured to be fixed to a head of the subject to hold the guide mirror (24) and the scanner (14) at a predetermined positional relationship with the eye of the subject without being influenced by movement of the head of the subject, wherein the scanner (14) and the guide mirror (24) are disposed on the guide mirror holder (10).

2. The scanning laser ophthalmoscope according to claim 1, wherein
the guide mirror (24) comprises a free-form surface or a combined structure of a free-form curve and a diffraction surface.

3. The scanning laser ophthalmoscope according to claim 1 or 2, wherein
the light source (12) is configured to be capable of simultaneously or selectively emitting first laser light (34a) and second laser light (34b), the first laser light (34a) comprising a wavelength in an infrared range used in acquiring the fundus image of the eye (22) of the subject, the second laser light (34b) comprising a wavelength in a visible range, and the light source (12) configured to irradiate the fundus of the eye (22) of the subject with the second laser light (34b) such that a photogene of the second laser light (34b) is recognized as a fixation target in a form of figure.

4. The scanning laser ophthalmoscope according to claim 3, wherein
the light source (12) is configured to be capable of changing a wavelength of laser light (34) to be emitted therefrom, and
the light source (12) is capable of selectively emitting the first laser light (34a) and the second laser light (34b) by controlling the wavelength of the laser light (34) to be emitted from the light source (12).

5. The scanning laser ophthalmoscope according to claim 3, wherein
the light source (12) comprises a first laser light emitter (121) configured to emit the first laser light (34a) and a second laser light emitter (122) configured to emit the second laser light (34b), and
the light source (12) is configured to be capable of simultaneously or selectively being in a state in which the first laser light (34a) is emitted from the first laser light emitter (121) and a state in which the second laser light (34b) is emitted from the second laser light emitter (122).

6. The scanning laser ophthalmoscope according to any one of claims 1 to 5, further comprising:
a split mirror (36) configured to split the reflected light of the laser light (34) reflected on the fundus,
wherein
the light receiver (38) is configured to receive the reflected light split by the split mirror (36),
the split mirror (36) is disposed at the guide mirror holder (10), and
the light receiver (38) is disposed at a position separated from the guide mirror holder (10).

## Patentansprüche

1. Scanning-Laser-Ophthalmoskop, umfassend:
eine Lichtquelle (12), die dazu konfiguriert ist, Laserlicht (34) zu emittieren;
einen Scanner (14), der dazu konfiguriert ist, das von der Lichtquelle (12) emittierte Laserlicht (34) zweidimensional abzutasten;
einen Führungsspiegel (24), der dazu konfiguriert ist, das von dem Scanner (14) abgetastete Laserlicht (34) zu einem Fundus eines Auges (22) eines Subjekts zu führen;
einen Lichtempfänger (38), der dazu konfiguriert ist, reflektiertes Licht des Laserlichts (34), das am Fundus reflektiert wird, zu empfangen; und
einen Bildgenerator (39), der dazu konfiguriert ist, ein Fundusbild auf der Grundlage des von dem Lichtempfänger (38) empfangenen reflektierten Lichts zu erzeugen,
wobei
der Führungsspiegel (24) auf einem Pfad angeordnet ist, der den Scanner (14) und den Fundus des Auges (22) des Subjekts verbindet, und vor dem Auge (22) des Subjekts angeordnet ist,
**gekennzeichnet durch** einen Führungsspiegelhalter (10), der eine Struktur umfasst, die so konfiguriert ist, dass sie an einem Kopf des Subjekts befestigt werden kann, um den Scanner (14) und den Führungsspiegel (24) in einer vorbestimmten Positionsbeziehung mit dem Auge (22) des Subjekts zu halten, ohne durch die Bewegung des Kopfes des Subjekts beeinflusst zu werden, wobei der Scanner (14) und der Führungsspiegel (24) an dem Führungsspiegelhalter (10) angeordnet sind.

2. Scanning-Laser-Ophthalmoskop nach Anspruch 1, wobei
der Führungsspiegel (24) eine Freiformfläche oder eine kombinierte Struktur aus einer Freiformkurve und einer Beugungsfläche aufweist.

3. Scanning-Laser-Ophthalmoskop nach Anspruch 1 oder 2, wobei
die Lichtquelle (12) so konfiguriert ist, dass sie in der Lage ist, gleichzeitig oder selektiv ein erstes Laserlicht (34a) und ein zweites Laserlicht (34b) zu emittieren, wobei das erste Laserlicht (34a) eine Wellenlänge in einem Infrarotbereich aufweist, der bei der Erfassung des Fundusbildes des Auges (22) des Subjekts verwendet wird, das zweite Laserlicht (34b) eine Wellenlänge in einem sichtbaren Bereich umfasst, und die Lichtquelle (12) so konfiguriert ist, dass sie den Fundus des Auges (22) des Subjekts mit dem zweiten Laserlicht (34b) bestrahlt, so dass ein Photogen des zweiten Laserlichts (34b) als ein Fixationsziel in Form einer Figur erkannt wird.

4. Scanning-Laser-Ophthalmoskop nach Anspruch 3, wobei
die Lichtquelle (12) so konfiguriert ist, dass sie in der Lage ist, eine Wellenlänge des von ihr zu emittierenden Laserlichts (34) zu ändern, und
die Lichtquelle (12) in der Lage ist, das erste Laserlicht (34a) und das zweite Laserlicht (34b) selektiv zu emittieren, indem die Wellenlänge des von der Lichtquelle (12) zu emittierenden Laserlichts (34) gesteuert wird.

5. Scanning-Laser-Ophthalmoskop nach Anspruch 3, wobei
die Lichtquelle (12) einen ersten Laserlichtemitter (121) umfasst, der dazu konfiguriert ist, das erste Laserlicht (34a) zu emittieren, und einen zweiten Laserlichtemitter (122), der dazu konfiguriert ist, das zweite Laserlicht (34b) zu emittieren, und
die Lichtquelle (12) so konfiguriert ist, dass sie in der Lage ist, gleichzeitig oder selektiv in einem Zustand zu sein, in dem das erste Laserlicht (34a) von dem ersten Laserlichtemitter (121) emittiert wird, und in einem Zustand, in dem das zweite Laserlicht (34b) von dem zweiten Laserlichtemitter (122) emittiert wird.

6. Scanning-Laser-Ophthalmoskop nach einem der Ansprüche 1 bis 5, ferner umfassend:
einen geteilten Spiegel (36), der so konfiguriert ist, dass er das reflektierte Licht des Laserlichts (34), das auf dem Fundus reflektiert wird, teilt,
wobei
der Lichtempfänger (38) dazu konfiguriert ist, das reflektierte Licht zu empfangen, das durch den geteilten Spiegel (36) geteilt wird,
der geteilte Spiegel (36) an dem Führungsspiegelhalter (10) angeordnet ist, und
der Lichtempfänger (38) an einer von dem Führungsspiegelhalter (10) getrennten Position angeordnet ist.

## Revendications

1. Ophtalmoscope laser à balayage comprenant :
une source de lumière (12) configurée pour émettre une lumière laser (34) ;
un dispositif de balayage (14) configuré pour balayer la lumière laser (34) émise à partir de la source de lumière (12) de manière bidimensionnelle ;
un miroir de guidage (24) configuré pour guider la lumière laser (34) balayée par le dispositif de balayage (14) vers un fond d'oeil d'un oeil (22) d'un sujet ;
un récepteur de lumière (38) configuré pour recevoir une lumière réfléchie de la lumière laser (34) réfléchie sur le fond d'oeil ; et
un générateur d'image (39) configuré pour générer une image de fond d'oeil sur la base de la lumière réfléchie reçue par le récepteur de lumière (38),
dans lequel
le miroir de guidage (24) est disposé sur un trajet reliant le dispositif de balayage (14) et le fond d'oeil de l'œil (22) du sujet, et disposé devant l'œil (22) du sujet,
**caractérisé par**
un support de miroir de guidage (10) comprenant une structure configurée pour être fixée à une tête du sujet pour soutenir le miroir de guidage (24) et le dispositif de balayage (14) dans une relation de position prédéterminée avec l'œil du sujet sans qu'ils soient influencés par un mouvement de la tête du sujet, dans lequel le dispositif de balayage (14) et le miroir de guidage (24) sont disposés sur le support de miroir de guidage (10).

2. Ophtalmoscope laser à balayage selon la revendication 1, dans lequel
le miroir de guidage (24) comprend une surface de forme libre ou une structure combinée d'une courbe de forme libre et d'une surface de diffraction.

3. Ophtalmoscope laser à balayage selon la revendication 1 ou 2, dans lequel
la source de lumière (12) est configurée pour être capable d'émettre simultanément ou sélectivement une première lumière laser (34a) et une seconde lumière laser (34b), la première lumière laser (34a) comprenant une longueur d'onde dans une gamme de l'infrarouge utilisée dans l'acquisition de l'image de fond d'oeil de l'œil (22) du sujet, la seconde lumière laser (34b) comprenant une longueur d'onde dans une gamme du visible, et la source de lumière (12) étant configurée pour éclairer le fond d'oeil de l'œil (22) du sujet avec la seconde lumière laser (34b) de sorte qu'un photogène de la seconde lumière laser (34b) soit reconnu comme un point de fixation sous une forme de figure.

4. Ophtalmoscope laser à balayage selon la revendication 3, dans lequel
la source de lumière (12) est configurée pour être capable de changer une longueur d'onde de lumière laser (34) devant être émise à partir de celle-ci, et
la source de lumière (12) est capable d'émettre sélectivement la première lumière laser (34a) et la seconde lumière laser (34b) en commandant la longueur d'onde de la lumière laser (34) devant être émise à partir de la source de lumière (12).

5. Ophtalmoscope laser à balayage selon la revendication 3, dans lequel
la source de lumière (12) comprend un premier émetteur de lumière laser (121) configuré pour émettre la première lumière laser (34a) et un second émetteur de lumière laser (122) configuré pour émettre la seconde lumière laser (34b), et
la source de lumière (12) est configurée pour être capable d'être simultanément ou sélectivement dans un état dans lequel la première lumière laser (34a) est émise à partir du premier émetteur de lumière laser (121) et un état dans lequel la seconde lumière laser (34b) est émise à partir du second émetteur de lumière laser (122).

6. Ophtalmoscope laser à balayage selon l'une quelconque des revendications 1 à 5, comprenant en outre :
un miroir de séparation (36) configuré pour séparer la lumière réfléchie de la lumière laser (34) réfléchie sur le fond d'oeil,
dans lequel
le récepteur de lumière (38) est configuré pour recevoir la lumière réfléchie séparée par le miroir de séparation (36),
le miroir de séparation (36) est disposé au niveau du support de miroir de guidage (10), et
le récepteur de lumière (38) est disposé à une position distincte du support de miroir de guidage (10).
